(19) Europäisches Patentamt
European Patent Office
Office européen des brevets

(11) Numéro de publication: **0 056 765**
**B1**

(12) **FASCICULE DE BREVET EUROPEEN**

(45) Date de publication du fascicule du brevet: **19.06.85**

(51) Int. Cl.[4]: **C 07 D 215/22**

(21) Numéro de dépôt: **82400079.8**

(22) Date de dépôt: **15.01.82**

(54) **Préparation d'hydroxy-4 quinoléines.**

(30) Priorité: **16.01.81 FR 8100764**

(43) Date de publication de la demande:
**28.07.82 Bulletin 82/30**

(45) Mention de la délivrance du brevet:
**19.06.85 Bulletin 85/25**

(84) Etats contractants désignés:
**AT BE CH DE FR GB IT LI LU NL SE**

(56) Documents cités:
**US-A-2 558 211**

**JOURNAL OF THE AMERICAN CHEMICAL SOCIETY, vol. 74, 20 septembre 1952, WASHINGTON (US), W.S. JOHNSON et al. "A new synthesis of chloroquine", pages 4513-16**

**Le dossier contient des informations techniques présentées postérieurement au dépôt de la demande et ne figurant pas dans le présent fascicule.**

(73) Titulaire: **RHONE-POULENC SANTE
Les Miroirs 18 Avenue d'Alsace
F-92400 Courbevoie Cedex (FR)**

(72) Inventeur: **Baudouin, Michel
78, avenue Parmentier
F-69190 St-Fons (FR)**
Inventeur: **Michelet, Daniel
24, Chemin de Montribloud
F-69160 Tassin (FR)**

(74) Mandataire: **Pilard, Jacques et al
RHONE-POULENC RECHERCHES Service
Brevets Pharma 25, Quai Paul Doumer
F-92408 Courbevoie Cedex (FR)**

**EP 0 056 765 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne un procédé de préparation d'hydroxy-4 quinoléines de formule générale (I):

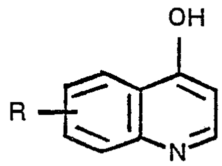

I

dans laquelle R représente 1 ou 2 substituants identiques ou différents choisis parmi les atomes d'halogène en position 5, 6, 7 ou 8 et les radicaux alcoyles contenant 1 à 4 atomes de carbone en position 2, 3, 5, 6, 7 ou 8, un au moins étant un atome d'halogène, à partir des tétrahydro-1,2,3,4 quinolinones-4 correspondantes de formule générale (II):

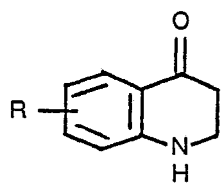

II

dans laquelle R est défini comme précédemment.

Les produits de formule générale (I) sont des intermédiaires particulièrement intéressants pour la synthèse de produits thérapeutiquement actifs, tels que la glafénine [ester dihydroxypropylique de l'acide N-(chloro-7 quinolyl-4) anthranilique] qui est un analgésique puissant, la chloroquine [chloro-7 (diéthylamino-4-méthyl-1 butylamino)-4 quinoléine] ou l'amodiaquine [chloro-7 (diéthylaminométhyl-3 hydroxy-4 phénylamino)-4 quinoléine] qui présentent des propriétés antimalariques remarquables.

Il est connu de préparer une hydroxy-4 quinoléine à partir de la quinolinone correspondante soit par deshydrogénation catalytique en présence de palladium sur charbon [W. S. JOHNSON et B. G. BUELL, J. Amer. Chem. Soc., *74*, 4513 (1952)], soit par transfert d'hydrogène catalysé par le palladium sur charbon en présence d'acide maléique (brevet des Etats-Unis d'Amérique 2 558 211). Cependant, lorsque l'on utilise une tétrahydro-1,2,3,4 quinolinone-4 de formule générale (II) dans laquelle R représente un atome d'halogène, l'aromatisation s'accompagne d'une déhalogénation ce qui conduit à l'obtention d'un mélange de l'hydroxy-4 quinoléine halogénée et de l'hydroxy-4 quinoléine.

Il a maintenant été trouvé, et c'est de qui fait l'objet de la présente invention que l'hydroxy-4 quinoléine de formule générale (I) peut être obtenue, avec de bons rendements, par oxydation, au moyen d'oxygène ou d'air, de la tétrahydro-1,2,3,4 quinolinone-4 correspondante en présence d'un catalyseur à base de platine ou de ruthénium sur charbon.

La réaction est effectuée en milieu aqueux basique en opérant à une température comprise entre 70°C et la température d'ébullition du mélange réactionnel et en agitant le mélange réactionnel en contact avec l'oxygène pur ou l'air. Par ailleurs, dans le but d'augmenter la vitesse de réaction, il est particulièrement avantageux d'opérer sous une pression qui peut atteindre 10 bars.

Le catalyseur contient, de préférence, environ 5% de métal et il est utilisé en quantité telle que le métal représente environ 0,01 atome-gramme par mole de tétrahydro-1,2,3,4 quinolinone-4 mise en oeuvre.

La mise en oeuvre du procédé selon l'invention sur une halogéno tétrahydro-1,2,3,4 quinolinone-4 permet d'obtenir l'halogeno-hydroxy-4 quinoléine correspondante pratiquement exempte d'hydroxy-4 quinoléine.

L'hydroxy-4 quinoléine de formule générale (I) obtenue selon la présente invention peut être séparée du mélange réactionnel et purifiée par application des méthodes hibituelles telles que la cristallisation ou la chromatographie.

La tétrahydro-1,2,3,4 quinolinone-4 de formule générale (II) utilisée comme produit de départ peut avantageusement être préparée par cyclisation de l'acide anilino-3 propionique correspondant au moyen d'un mélange d'acide fluorhydrique et de trifluorure de bore.

L'acide anilino-3 propionique peut être obtenu par action d'un excès d'aniline convenablement substituée sur l'acide acrylique. Généralement, la réaction est effectuée dans l'eau à une température comprise entre 70 et 100°C. La durée de la réaction est généralement comprise entre 1 et 4 heures.

Par exemple, la chloro-7 hydroxy-4 quinoléine obtenue selon le procédé de la présente invention peut être transformée en glafénine selon le procédé décrit dans le brevet français 2413 M après transformation en dichloro-4,7 quinoléine au moyen, par exemple, d'oxychlorure de phosphore.

Les exemples suivants, montrent comment l'invention peut être mise en pratique.

Exemple 1

Un ballon de 100 cm3, équipé d'un agitateur magnétique et d'un réfrigérant ascendant est placé dans un bain d'huile chauffant. Une tubulure fermée par un verre fritté permet d'introduire l'air dans la phase liquide.

Dans le ballon, on charge successivement:

— 2,7032 g de chloro-7 tétrahydro-1,2,3,4 quinolinone-4 (14,9 . $10^{-3}$ M)

— 0,538 g d'un catalyseur constitué de platine sur charbon (5% en poids)

— 30 cm3 de soude

Après avoir chargé les réactifs, le contenu du ballon est chauffé au reflux et balayé par de l'air pendant toute la réaction. Après 6 heures de chauffage, le mélange réactionnel est refroidi.

Le catalyseur est séparé par filtration et lavé sur le filtre avec du chlorure de méthylène. Le filtrat est extrait 2 fois avec 100 cm3 de chlorure de méthylène. Les phases chlorométhyléniques sont réunies et évaporées à sec. On recueille 36 mg de chloro-7 tétrahydro-1,2,3,4 quinolinone-4 contenant des traces de chloro-7 hydroxy-4 quinoléine.

La phase aqueuse est ajustée à pH 6,0 par addition d'acide sulfurique N puis elle est extraite par 2 fois 100 cm3 de butanol normal.

Après évaporation du butanol sous pression réduite, on obtient 2,57 g de chloro-7 hydroxy-4 quinoléine pratiquement pure.

La chloro-7 hydroxy-4 quinoléine est obtenue avec:

un taux de transformation de la chloro-7 tétrahydro-1,2,3,4 quinolinone-4 de 98,7%

un rendement en chloro-7 hydroxy-4 quinoléine de 96,2% par rapport à la chloro-7 tétrahydro-1,2,3,4 quinolinone transformée.

La chloro-7 tétrahydro-1,2,3,4 quinolinone-4 de départ peut être préparé de la manière suivante:

Dans un réacteur en acier inoxydable contenant 50 g d'acide fluorhydrique liquide refroidi à 5°C on ajoute 10 g d'acide m-chloroanilino-3 propionique (titrant 94,5%).

La solution est saturée de trifluorure de bore gazeux. A cet effet, le contenu du réacteur est maintenu à 20°C puis saturé par le trifluorure de bore gazeux sous une pression de 13 bars pendant 1 heure. Le réacteur est ensuite fermé puis chauffé à 80°C pendant 20 heures.

La pression s'élève d'abord jusqu'à 20 bars puis diminue progressivement pour se stabiliser vers 16 bars. Le réacteur est ensuite refroidi à 10°C puis ouvert de manière à laisser échapper le trifluorure de bore. Le liquide rougeâtre obtenu est versé dans un mélange d'eau et de glace. Après extraction 3 fois par 100 cm3 de chloroforme, la couche organique est lavée plusieurs fois par 100 cm3 d'eau jusqu'à pH compris entre 3 et 4 puis séchée sur sulfate de sodium. Après filtration et concentration à sec sous pression réduite (10 mm de mercure; 1,33 kPa), on obtient 9 g de chloro-7 tétrahydro-1,2,3,4 quinolinone-4 cristallisée dont le titre déterminé par chromatographie en phase gazeuse est 94,5%.

Le taux de transformation est de 100% et le rendement par rapport à l'acide m-chloroanilino-3 propionique est de 99%.

Par examen en chromatographie en phase gazeuse, la teneur en isomère chloro-5 est voisine de 0,7%.

L'acide m-chloroanilino-3 propionique de départ peut être préparé de la manière suivante:

A un mélange de 510,3 g de m-chloroaniline dans 150 cm3 d'eau, maintenu sous atmosphère d'argon et agité à 80°C, on ajoute en 10 minutes une solution de 72,05 g d'acide acrylique dans 100 cm3 d'eau. Le mélange réactionnel constitué de 2 phases est maintenu pendant 3 heures à 80°C sous agitation puis refroidi à 20°C. Après décantation, la phase aqueuse (couche supérieure) est éliminée. A la phase organique on ajoute 423 cm3 d'une solution aqueuse de soude 2,6 N, en agitant et en maintenant la température à 20°C. Après décantation, la phase organique constituée de 303 g de m-chloroaniline est séparée. La phase aqueuse (850 cm3) est extraite 6 fois successivement par 450 cm3 d'éther.

La phase aqueuse, dont on élimine l'éther par évaporation sous pression réduite (20 mm de mercure; 2,7 kPa), est acidifiée par addition de 105 g d'acide sulfurique à 50% (en poids). Le pH final est 3,5 (point isoélectrique). La température passe de 22 à 33°C puis on chauffe à 40°C. Après décantation, on sépare:

une phase organique inférieure (208,8 g) constituée d'acide m-chloroanilino-3 propionique fondu, saturé d'eau (8,6% d'eau)

une phase aqueuse supérieure (601 g) contenant 2,28 g d'acide m-chloroanilino propionique et 156 g de sulfate de sodium.

La phase organique est chauffée pendant 1 heure à 80°C sous pression réduite (20 mm de mercure; 2,7 kPa). On obtient 195,4 g d'un produit contenant 94% d'acide m-chloroanilino-3 propionique et 2,3% d'eau.

Exemple 2

Un ballon de 100 cm3 équipé d'un agitateur magnétique et d'un réfrigérant ascendant est placé dans un bain d'huile chauffant. Le sommet du réfrigérant est relié à un gazomètre permettant de mesurer le volume de gaz absorbé.

Dans le ballon, on charge successivement:

catalyseur composé de 50% en poids de ruthenium sur charbon et de 50% d'eau (la teneur en ruthenium dans le catalyseur sec est de 5% poids/poids) . . . . . . . . . . . . . . . . . . . . . 0,6130 g

chloro-7 tétrahydro-1,2,3,4 quinolinone-4 . . . . . . 1,6089 g

soude N (en solution aqueuse) . . . . . . . . . . . . .60 cm3

On purge l'appareil avec de l'oxygène pur, remplit le gazomètre d'oxygène et chauffe pendant 7 heures 15 minutes la masse réactionnelle à 70°C. Le volume d'oxygène absorbè est de 27 cm3 (théorie 111 cm3).

On refroidit, sépare le catalyseur par filtration et extrait par 2 fois 50 cm3 de chlorure de méthylène.

On dose 0,944 g de chloro-7 tétrahydro-1,2,3,4 quinolinone-4 dans l'extrait chlorométhylénique ce qui correspond à un taux de transformation de 41,3%.

La solution aqueuse basique est ajustée à pH 6 par addition d'acide sulfurique N puis extraite par 2 fois 50 cm3 de butanol normal. Le butanol est évaporé sous pression réduite. On dose ainsi par chromatographie gazeuse 0,473 g de dichloro-4,7 quinoléine correspondant à un rendement de 65% en hydroxy-4 chloro-7 quinoléine par rapport à la chloro-7 tétrahydro-1,2,3,4 quinolinone-4 transformée.

Par examen chromatographique on ne détecte pas de chloro-4 quinoléine.

Exemple 3

On opère comme dans l'exemple 2 mais en utilisant les produits suivants:

platine sur charbon à 5% (p/p). . . . . . . . . . . . . 0,3266 g

chloro-7 tétrahydro-1,2,3,4 quinolinone-4 . . . . . . 1,5477 g

solution aqueuse de soude N . . . . . . . . . . . . . .60 cm3

Après avoir purgé l'appareil avec de l'oxygène pur on chauffe à 70°C pendant 7 heures 15 minutes. Le volume d'oxygène absorbé est de 37 cm3 (théorie 106,5 cm3).

Le mélange réactionnel est traité dans les conditions de l'exemple 2.

On dose ainsi 0,925 g de chloro-7 tétrahydro-1,2,3,4 quinolinone-4 non convertie correspondant à un taux de transformation de 40,3%.

La solution aqueuse basique est traitée comme à l'exemple 2. On dose par chromatographie gazeuse 0,602 g de dichloro-4,7 quinoléine correspondant à un rendement de 88,6% en hydroxy-4 chloro-7 quinoléine par rapport à la chloro-7 tétrahydro-1,2,3,4 quinolinone-4 transformée. On ne détecte pas de chloro-4 quinoléine par chromatographie gazeuse.

Exemple 4

Dans un autoclave chemisé par du Teflon de capacité 140 cm3 on charge:

platine à 5% sur charbon (p/p). . . . . . . . . . . . . 0,3032 g

chloro-7 tétrahydro-1,2,3,4 quinolinone-4 . . . . . . 1,5106 g

solution aqueuse de soude N . . . . . . . . . . . . . .60 cm3

On place l'appareil sous une pression d'oxygène pur de 10 bars et chauffe à 70°C. La pression observée est alors de 11 bars; la pression diminue et se stabilise à 8,8 bars après 20 minutes. Après 32 minutes on refroidit et traite le mélange réactionnel dans les conditions décrites à l'exemple 2.

Le taux de transformation de la chloro-7 tétrahydro-1,2,3,4 quinolinone-4 est de 99,5%. On dose par chromatographie gazeuse, 1,105 g de dichloro-4,7 quinoléine correspondant à un rendement de 67,3% en hydroxy-4 chloro-7 quinoléine par rapport à la chloro-7 tétrahydro-1,2,3,4 quinolinone-4 transformée.

Exemple 5

Dans un appareil identique à celui utilisé dans l'exemple 1, on charge:

catalyseur décrit dans l'exemple 1 . . . . . . . . . . .0,531 g

méthyl-2 chloro-7 tétrahydro-1,2,3,4 quinolinone-4 . .2,940 g ($15 \times 10^{-3}$M)

soude N. . . . . . . . . . . . . . . . . . . . . . . . .30 cm3

$H_2O$ . . . . . . . . . . . . . . . . . . . . . . . . . . .75 cm3

On purge l'appareil avec de l'oxygène pur, remplit le gazomètre d'oxygène et chauffe pendant 2 heures la masse réactionnelle à 94°C. Le volume d'oxygène absorbé est de 165 cm3 (théorie 168 cm3).

La masse réactionnelle est refroidie. Le catalyseur est séparé par filtration et lavé sur le filtre avec du chlorure de méthylène. Les phases chlorométhylèniques sont réunies et évaporées à sec. On recueille 0,807 g de méthyl-2 chloro-7 tétrahydro-1,2,3,4 quinolinone-4 contenant des traces de méthyl-2 hydroxy-4 chloro-7 quinoléine.

La phase aqueuse est ajustée à pH 6.0 par addition d'acide sulfurique N, puis est extraite avec deux fois 100 cm3 de butanol normal. Le butanol est évaporé sous pression réduite. On obtient ainsi 1,867 g de méthyl-2 hydroxy-4 chloro-7 quinoléine.

Le méthyl-2 hydroxy-4 chloro-7 quinoléine est obtenu avec:

un taux de transformation de 72% de la méthyl-2 chloro-7 tétrahydro-1,2,3,4 quinolinone,

un rendement en méthyl-2 hydroxy-4 chloro-7 quinoléine de 89% par rapport à la méthyl-2 chloro-7 tétrahydro-1,2,3,4 quinolinone transformée.

**Revendications**

1. Procédé de préparation d'une hydroxy-4 quinoléine de formule générale (I):

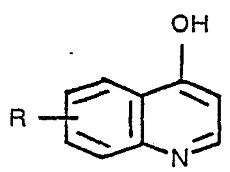

I

dans laquelle R représente 1 ou 2 substituants identiques ou différents choisis parmi les atomes d'halogène en position 5, 6, 7 ou 8 radicaux alcoyles contenant 1 à 4 atomes de carbone en position 2, 3, 5, 6, 7 ou 8, un au moins étant un atome d'halogène, caractérisé en ce que l'on oxyde une tétrahydro-1,2,3,4 quinolinone-4 de formule générale (II):

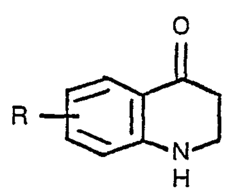

II

dans laquelle R est défini comme précédemment, au moyen d'oxygène ou d'air en présence d'un catalyseur à base de platine ou de ruthénium sur charbon, à raison de 0,01 atome-gramme environ de métal par mole de quinolinone, à une température comprise entre 70°C et la température d'ébullition du mélance réactionnel, en opérant en milieu aqueux basique.

2. Procédé selon la revendication 1 caractérisé en ce que l'on opère sous pression.

3. Procédé selon la revendication 1 ou 2 pour la préparation de la chloro-7 hydroxy-4 quinoléine pratiquement exempte d'hydroxy-4 quinoléine caractérisé en ce que l'on oxyde la chloro-7 tétrahydro-1,2,3,4 quinolinone-4.

**Patentansprüche**

1. Verfahren zur Herstellung von einem 4-hydroxychinolin der allgemeinen Formel (I):

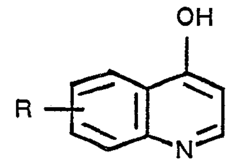

I

worin R einen oder zwei gleiche oder verschiedene unter den Halogenatomen in 5-, 6-, 7- oder 8-Stellung und den Alkylresten mit 1—4 Kohlenstoffatomen in 2-, 3-, 5-, 6-, 7- oder 8-Stellung ausgewählte Substituenten bedeutet, wobei mindestens einer ein Halogenatom ist, dadurch gekennzeichnet, daß man ein 1,2,3,4-Tetrahydrochinolin-4-on der allgemeinen Formel (II):

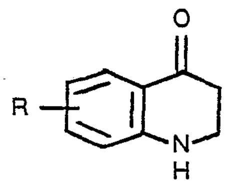

II

worin R wie oben definiert ist, mittels Sauerstoff oder Luft in Gegenwart eines Katalysators auf Basis von Platin oder Ruthenium auf Kohle in einer Menge von etwa 0,01 Grammatom Metall pro Mol Chinolinon bei einer Temperatur zwischen 70°C und der Siedetemperatur der Reaktionsmischung oxidiert, wobei man in wässerigem basischem Milieu arbeitet.

2. Verfahren nach Anspruch 1, dadurch gekennzeichnet, daß man unter Druck arbeitet.

3. Verfahren nach Anspruch 1 oder 2 zur Herstellung von 7-Chlor-4-hydroxychinolin, das praktisch frei von 4-Hydroxychinolin ist, dadurch gekennzeichnet, daß man das 7-Chlor-1,2,3,4-tetrahydrochinolin-4-on oxidiert.

**Claims**

1. Process for the preparation of a 4-hydroxy-quinoline of the general formula (I):

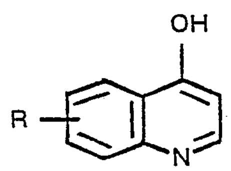

I

in which R represents 1 or 2 identical or different substituents chosen from among halogen atoms in position 5, 6, 7 or 8 and alkyl radicals, containing 1 to 4 carbon atoms, in position 2, 3, 5, 6, 7 or 8, at least one substituent being a halogen atom, characterised in that a 1,2,3,4-tetrahydro-quinolin-4-one of the general formula (II):

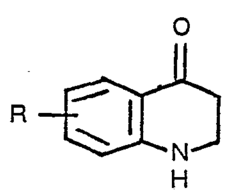

II

in which R is defined as above, is oxidised by means of oxygen or air in the presence of a catalyst based on platinum or ruthenium on charcoal, using about 0.01 gram-atom of metal per mole of quinolinone, at a temperature between about 70°C and the boiling point of the reaction mixture, in a basic aqueous medium.

2. Process according to Claim 1, characterised in that it is carried out under pressure.

3. Process according to Claim 1 or 2 for the preparation of 7-chloro-4-hydroxy-quinoline virtually free from 4-hydroxy-quinoline, characterised in that 7-chloro-1,2,3,4-tetrahydro-quinolin-4-one is oxidised.